# EUROPEAN PATENT APPLICATION

(11) **EP 4 431 613 A1**
(43) Date of publication of application: **18.09.2024**
(21) Application number: 23161782.0
(22) Date of filing: 14.03.2023
(51) Int. Cl.: C12P 19/02, C12P 19/24, C12N 9/90

(54) **METHOD FOR THE PRODUCTION OF D-TAGATOSE USING AN L-ARABINOSE ISOMERASE FROM LENTILACTOBACILLUS PARAKEFIRI**

(71) Applicant: Universität Hohenheim, 70599 Stuttgart (DE)
(72) Inventor: Fischer, Lutz, 73274 Notzingen (DE); Weber, Nathanael, 70599 Stuttgart (DE); Lutz-Wahl, Sabine, 73230 Kirchheim unter Teck (DE)
(74) Representative: Müller-Boré & Partner Patentanwälte PartG mbB

(57) **Abstract**

The present invention relates to methods for the production of D-tagatose, said methods using an L-arabinose isomerase enzyme derived from *Lentilactobacillus parakefiri* and D-galactose as substrate for isomerization under acidic conditions using sour whey as lactose source to be hydrolysed to galactose.

## Description

The present invention relates to methods for the production of D-tagatose, said methods using an L-arabinose isomerase enzyme derived from *Lentilactobacillus parakefiri,* as well as respective products containing D-tagatose.

The natural sugar tagatose is a rare simple sugar with increasing commercial interest as a sweetener. This is because tagatose has comparable sweetening power to sucrose, but only about a three-quarter of its calories. Due to the very slow resorption of tagatose, there is also only a slight increase in blood sugar, which makes the sugar very suitable for diabetics. In addition, the sugar is considered tooth-friendly because it is not metabolized by caries-causing bacteria and thus does not contribute to acid formation in the mouth.

Tagatose can be used wherever conventional household sugar was previously used. The advantage is that tagatose, with its strong sweetening power, only needs to be dosed half as much as some other sugar alternatives. The particularly low glycemic index of 3 of tagatose (compared to 100 for glucose) also makes the sugar interesting for use in dietary foods, especially for diabetics. In addition, tagatose is tooth-friendly because it is not metabolized by caries-causing bacteria and thus does not contribute to acid formation in the mouth, therefore not causing caries. Further, tagatose is prebiotic, *i.e*., promotes microorganisms in the intestine. Accordingly, tagatose is an ideal substitute for the conventional sweeteners glucose, fructose or other sugar substitutes.

Despite these advantageous properties, tagatose has so far only been able to establish itself in individual niche products and not on a broad basis in food technology. The main reason for this niche existence is that the chemical processes for producing tagatose are complex and, therefore, expensive. Alternatives to the chemical process are biotechnological approaches using the enzyme L-arabinose isomerase (L-AI). Here, the enzyme converts the commercially available simple sugar galactose into the related sugar tagatose by rearranging certain chemical groups. However, this is not yet economically feasible in view of the approximately 90 US$ production costs per kilogram.

In recent years, L-AI from different organisms have been found and investigated in order to further increase the effectiveness of tagatose production. However, none of these enzymes has yet reached market maturity.

The cheapest source of galactose is lactose, an abundant, cheap by-product derived from whey. Whey is a by-product of curd and cheese production, which is produced during the curdling of milk (*i.e.*, the coagulation of the milk casein). Large quantities of sweet whey with a pH value greater than 5.5 (when using rennet) or sour whey with a pH value less than 5.2 (when using lactic acid bacteria) are produced. Due to the acidic pH value, sour whey cannot be further processed and must ultimately be disposed of unused.

The dairy industry in Germany currently uses around 4 million dairy cows in around 54,000 dairy farms, producing over 30 million tons of milk per year. From this quantity of milk, the various dairy products such as yoghurt, curd, cheese, etc. are produced, resulting in huge quantities of whey. If the yellowish, watery residue from the milk has an almost neutral pH value of 5.5 to 6.5, it is called sweet whey and can still be further processed - for example as protein powder for athletes, pet food, wellness drinks or in cosmetics. However, as soon as lactic acid bacteria are involved in the production of dairy products, sour whey with a pH value of around 4 and a lower protein content is produced, so that further processing is uneconomical, while disposal is expensive and energy-intensive. In 2021, around 13 million tons of whey were produced in Germany as a waste product that could not be further processed. The problem here is that whey is relatively difficult to dispose of, as whey cannot simply be thrown away due to legal disposal guidelines. Accordingly, the dairies have the problem of disposing of the whey in compliance with the law, which is often expensive and time-consuming. In limited quantities, the whey can be used by farmers as fertilizer and animal feed, but not to the extent of absorbing the entire volume of whey.

To date, there is no established process or application to make sensible use of the large quantities of whey in Germany (and worldwide). The existing solutions are often too expensive, too complex, not suitable for large-scale implementation or only focused on a niche market.

Commercially used processes for the enzymatic production of tagatose use lactose as a starting material, which is hydrolyzed into glucose and galactose. Subsequently, L-arabinose isomerase variants of *Geobacillus stearothermophilus* or *Thermotoga neapolitana* are used for the isomerization of galactose. This is done at a neutral pH of pH 6.5 to pH 7.5, since these L-arabinose isomerases have low activity and stability at a pH below pH 6.5. Therefore, these enzymes are not suitable for the isomerization of galactose in sour whey, which has a pH of less than pH 5.2.

Accordingly, the technical problem underlying the present invention is the provision of means for the enzymatic production of tagatose, preferably using lactose as a starting material, wherein said lactose is preferably whey, more preferably sour whey.

The solution to the above technical problem is achieved by the embodiments characterized in the claims.

In particular, in a fist aspect, the present invention relates to a method for the production of D-tagatose, comprising the steps of:
(a) providing a solution containing D-galactose;
(b) adding an enzyme having L-arabinose isomerase activity to the solution provided in step (a), wherein said enzyme comprises
   (i) the amino acid sequence of SEQ ID NO: 1; or
   (ii) an amino acid sequence having at least 70% sequence identity to SEQ ID NO: 1 and having L-arabinose isomerase activity; and
(c) incubating the solution with the added enzyme having L-arabinose isomerase activity under conditions and for a duration of time allowing for the enzymatic isomerization of the D-galactose to D-tagatose by said enzyme having L-arabinose isomerase activity.

In step (a) of the method of the present invention, a solution containing D-galactose is provided. This solution is not particularly limited and includes any conceivable solution containing D-galactose. However, in preferred embodiments of the present invention, said D-galactose is derived from the enzymatic cleavage of lactose.

Thus, in these embodiments, step (a) of the method of the present invention preferably comprises the steps of:
(α-1) providing a solution containing lactose;
(α-2) adding an enzyme having β-galactosidase activity to the solution provided in step (α-1); and
(α-3) incubating the solution with the added enzyme having β-galactosidase activity under conditions and for a duration of time allowing for the enzymatic cleavage of the lactose into D-glucose and D-galactose by said enzyme having β-galactosidase.

In the above step (α-1), a solution containing lactose is provided. This solution is not particularly limited and includes any conceivable solution containing lactose. However, in preferred embodiments of the present invention, the solution containing lactose provided in step (α-1) is whey, preferably either (i) sweet whey, having a pH of at least pH 5.5, preferably a pH in the range of about pH 5.5 to pH 6.8, or (ii) sour whey, having a pH of less than pH 5.2, preferably a pH in the range of about pH 4.0 to about pH 5.2, wherein sour whey is particularly preferred. Preferably, the whey used in this respect, either sweet whey or sour whey, is unmodified whey without any previous separation of sugars or other compounds or components from said whey. Further, the solution containing lactose can be a solution generated from whey, i.e. generated from sour whey or sweet whey as defined above, wherein a solution generated from sour whey is particularly preferred. Solutions generated from whey are not particularly limited and include e.g. concentrates and filtrates of whey.

In the above step (α-2), an enzyme having β-galactosidase activity is added to the solution provided in step (α-1). Enzymes having β-galactosidase activity are not particularly limited and are known in the art. They include β-galactosidases (enzyme commission number EC 3.2.1.23), e.g. bacterial β-galactosidases, known in the art. By way example, at acidic pH values of e.g. pH 4.5, β-galactosidase from *Aspergillus niger* (e.g. marketed as MAXILACT^{®} A4) can be used, whereas at more neutral pH of e.g. pH 6.5, β-galactosidase from *Bacillus licheniformis* (e.g. marketed as Saphera^{®} 2600 L) or *Paenibacillus wynnii* can be used. Further, CelB from *Pyrococcus furiosus* can be used, e.g. at high temperatures of 55 to 80°C and a pH of 5.5 to 6.5.

In the above step (α-3), the solution with the added enzyme having β-galactosidase activity is incubated under conditions and for a duration of time allowing for the enzymatic cleavage of the lactose into D-glucose and D-galactose by said enzyme having β-galactosidase. Respective conditions and durations of time are not particularly limited and are known to the person skilled in the art and/or can be easily determined by the person skilled in the art. These include for example incubation at a temperature of from about 5 °C to about 40 °C, preferably of from about 10 °C to about 20 °C, e.g. at about 10 °C, at about 15 °C, or at about 20 °C. Further, suitable conditions include for example incubation at an acidic pH, preferably at a pH in the range of from about pH 4.5 to about pH 6.5. Furthermore, suitable durations of time can be easily determined by the person skilled in the art and include for example incubation for about 3 to 24 hours, e.g. for about 24 hours. Moreover, the amount of β-galactosidase activity that needs to be added in order to ensure enzymatic cleavage of the lactose into D-glucose and D-galactose can be easily determined by the person skilled in the art. Suitable amounts of β-galactosidase activity are known and/or can be determined by the person skilled in the art. By way of example, in the case of an *Aspergillus niger* β-galactosidase, 110 nkat_{Lactose}/mLₚₑᵣₘₑₐₜₑ can be used at a pH of 4.5 and a temperature of 15°C. Further, in the case of a *Bacillus licheniformis* β-galactosidase, 20 nkat_{Lactose}/mLₚₑᵣₘₑₐₜₑ can be used at a pH of 6.5 and a temperature of 15°C.

In step (b) of the method of the present invention, an enzyme having L-arabinose isomerase (L-AI) activity (enzyme commission number EC 5.3.1.4) is added to the solution provided in step (a), wherein said enzyme comprises
(i) the amino acid sequence of SEQ ID NO: 1; or
(ii) an amino acid sequence having at least 70% sequence identity to SEQ ID NO: 1 and having L-arabinose isomerase activity.

The enzyme having L-AI activity used in the present invention is (i) the L-AI from *Lentilactobacillus parakefiri* DSM 10551, the amino acid sequence of which is given in SEQ ID NO: 1, or (ii) and enzyme derived therefrom, having an amino acid sequence having at least 70% sequence identity to SEQ ID NO: 1 and having L-arabinose isomerase activity. The *Lentilactobacillus parakefiri* DSM 10551 L-AI is natively encoded by the nucleotide sequence of SEQ ID NO: 2. A nucleotide sequence encoding *Lentilactobacillus parakefiri* DSM 10551 L-AI and being optimized for expression in *E*. *coli* is given as SEQ ID NO: 3.

In preferred embodiments, the enzyme having L-AI activity consists of
(i) the amino acid sequence of SEQ ID NO: 1; or
(ii) an amino acid sequence having at least 70% sequence identity to SEQ ID NO: 1 and having L-arabinose isomerase activity.

In a particularly preferred embodiment, the enzyme having L-AI activity consists of the amino acid sequence of SEQ ID NO: 1.

The term "enzyme comprising an amino acid sequence having at least 70% sequence identity to SEQ ID NO: 1 and having L-arabinose isomerase activity" as used herein relates to polypeptides that can comprise any number of amino acid substitutions, additions, or deletions with respect to the amino acid sequence of SEQ ID NO: 1, e.g. 1, 2, 3, 4, 5, 6, 7, 8, 9, 10, or any number up to 150 (i.e., any integer n, wherein 1 ≤ n ≤ 150), amino acid substitutions, additions, or deletions with respect to the amino acid sequence of SEQ ID NO: 1, provided that the resulting polypeptides fulfil the requirement of having at least 70% sequence identity to SEQ ID NO: 1 and retain biological activity of an L-AI. In this context, the term "retains the biological activity of an L-AI" as used herein relates to polypeptides that have at least 5%, at least 10%, at least 15%, at least 20%, at least 25%, at least 30%, at least 35%, at least 40%, at least 45%, at least 50%, preferably at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 97.5%, at least 98%, at least 98.5%, at least 99%, at least 99.5%, 100%, or more than 100% of the activity of the *Lentilactobacillus parakefiri* DSM 10551 L-AI having the amino acid sequence of SEQ ID NO: 1.

While the number of amino acid substitutions, additions, or deletions is generally only limited by the above proviso concerning the sequence identity, biological activity of the resulting polypeptide, and absolute number of amino acid substitutions, additions or deletions, it is preferable that the resulting polypeptide has at least 72%, at least 74%, at least 76%, at least 78%, at least 80%, at least 82%, at least 84%, at least 86%, at least 88%, at least 90%, at least 91%, at least 92%, at least 93%, at least 94%, at least 95%, at least 95.5%, at least 96%, at least 96.5%, at least 97%, at least 97.5%, at least 98%, at least 98.2%, at least 98.4%, at least 98.6%, at least 98.8%, at least 99%, at least 99.1%, at least 99.2%, at least 99.3%, at least 99.4%, at least 99.5%, at least 99.55%, at least 99.6%, at least 99.65%, at least 99.7%, at least 99.75%, at least 99.8%, at least 99.85%, at least 99.9%, at least 99.91%, at least 99.92%, at least 99.93%, at least 99.94%, at least 99.95%, at least 99.96%, at least 99.97%, at least 99.98%, or at least 99.9% sequence identity to SEQ ID NO: 1.

Means for determining the sequence identity of an amino acid sequence to a reference sequence are known in the art.

In step (c) of the method of the present invention, the solution with the added enzyme having L-arabinose isomerase activity is incubated under conditions and for a duration of time allowing for the enzymatic isomerization of the D-galactose to D-tagatose by said enzyme having L-arabinose isomerase activity. Respective conditions and durations of time are not particularly limited and are known to the person skilled in the art and/or can be easily determined by the person skilled in the art. These include for example incubation at a temperature of from about 55 °C to about 70 °C, preferably of from about 60 °C to about 70 °C, e.g. at about 60 °C, at about 65 °C, or at about 70 °C. Further, suitable conditions include for example incubation at an acidic pH, preferably at a pH in the range of from about pH 4.5 to about pH 6.9, more preferably in the range of from about pH 4.5 to about pH 6.5. Furthermore, suitable durations of time can be easily determined by the person skilled in the art and include for example incubation for about 3 to 24 hours, e.g. for about 24 hours. Moreover, the amount of L-AI activity that needs to be added in order to ensure enzymatic isomerization of the D-galactose to D-tagatose can be easily determined by the person skilled in the art, and can e.g. be as defined above.

In certain embodiments, the method of the present invention further comprises a step of isolating D-tagatose from the solution obtained in step (c). Respective methods for the isolation of D-tagatose from a solution containing the same are not particularly limited and are known in the art.

In a second aspect, the present invention relates to a product containing D-tagatose, wherein said product is obtainable by the method according to the first aspect of the present invention.

In specific embodiments, the product is selected from the group consisting of foodstuffs, feedstuffs, pharmaceuticals, and food supplements, wherein the product is preferably a sweetener or a sugar syrup.

As used herein, the term "comprising"/"comprises" expressly includes the terms "consisting essentially of"/"consists essentially of" and "consisting of"/"consists of", *i.e.*, all of said terms are interchangeable with each other herein.

Further, as used herein, the term "about" represents a modifier of ± 10% of the specified value, preferably ± 7.5%, ± 5%, ± 3%, ± 2%, or ± 1% of the specified value. Thus, by way of example, the term "about 10" includes the ranges 9 to 11, 9.25 to 10.75, 9.5 to 10.5, 9.7 to 10.3, 9.8 to 10.2, and 9.9 to 10.1.

The present invention is based on the discovery of a novel L-arabinose isomerase (L-AI) from *Lentilactobacillus parakefiri* which can be used for the production of D-tagatose from D-galactose. The L-AI used in the present invention is an endogenous enzyme of said bacterial strain which can carry out the isomerization of galactose to tagatose under acidic pH conditions. This is a decisive advantage of the enzyme over other known versions of L-AI, which can usually only carry out the galactose-tagatose conversion under neutral or slightly alkaline pH values. With the new, acid-stable L-AI, this opens up new fields of application, especially with regard to sour whey and acidic galactose-containing food matrices, respectively. In particular, using the novel L-AI according to the present invention, an enzyme is now available that can be used for the processing of sour whey.

The present invention can be used e.g. in a bi-enzymatic *in situ* bioconversion of sour whey without first separating certain sugars or other components or changing the whey in any other way. In this case, the enzyme β-galactosidase is added to the sour whey, which almost completely splits the disaccharide lactose present in the sour whey into the single sugars glucose and galactose. With L-AI, which is also added according to the invention, the conversion of galactose to tagatose then takes place, although this conversion cannot take place completely for reasons of equilibrium. The enzyme reactions can take place e.g. in a pH range between about pH 4.5 and about 6.5 and at a temperature of about 60°C. Here, galactose can be isomerized to tagatose with a yield of about 50%, which corresponds to the maximum yield of the isomerization reaction. After completion of the reaction, the processed sour whey then contains a mixture of glucose, galactose and tagatose. This syrupy composition could be used as a sweetener, with the syrup containing fewer calories while maintaining sweetness. The proposed approach is also suitable for sweet whey. Thus, the present invention provides a new method for converting the vast amounts of unused whey into a product of interest in food technology.

The figures show:
Figure 1:
   Effect of pH on activity of L-arabinose isomerase of *Lentilactobacillus parakefiri.* The optimum pH was determined using four buffer systems in 100 mM: sodium acetate (pH 4.0-5.5, indicated in circle), MES (pH 5.5-6.5, indicated in square), MOPS (pH 6.5-7.9, indicated in triangle) and Bicine (pH 8-9, indicated in diamond), at 60 °C for 6 min with 300 mM D-galactose as substrate and 1.5 mM CoCl₂ as cofactor.
Figure 2:
   Isomerization of galactose (a, b) and glucose (b) in skim milk ultrafiltration permeate pH 4.5 and 6.5.
   After complete hydrolysis of 95 g/L lactose in skim milk ultrafiltration permeate pH 4.5 (A) and 6.5 (B) by β-galactosidase, galactose was isomerized by L-arabinose isomerase of *Lentilactobacillus parakefiri.* In addition, glucose was simultaneously isomerized by a glucose isomerase in skim milk ultrafiltration permeate pH 6.5 (b). The reactions were performed at 60 °C for 24 h.

The present invention will be further illustrated by the following examples without being limited thereto.

### Examples

### Example 1:

### Recombinant production of L-AI from Lentilactobacillus parakefiri DSM 10551

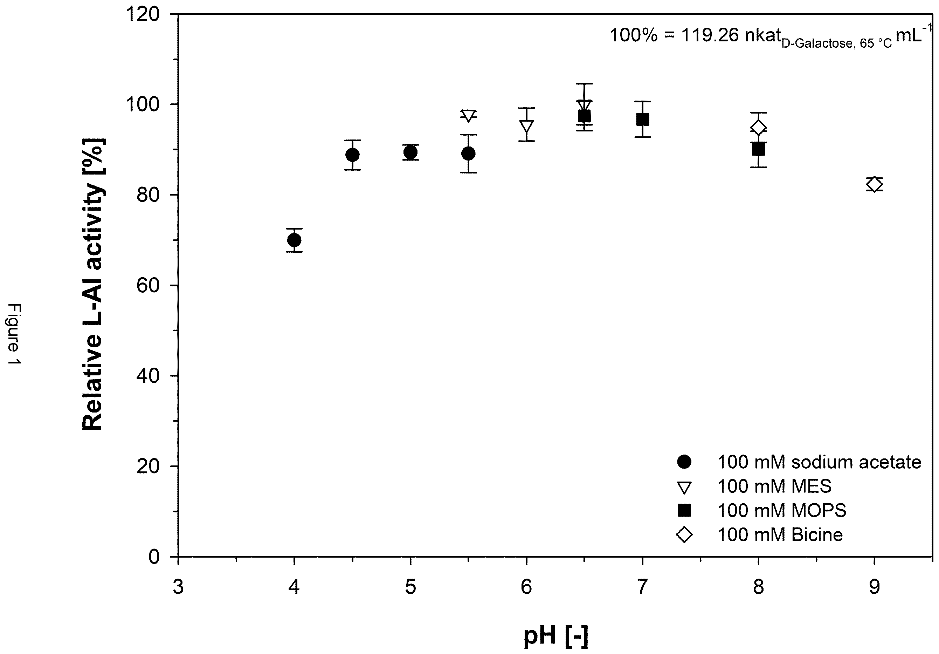

A novel, uncharacterized L-AI from *Lentilactobacillus parakefiri* DSM 10551 was produced recombinantly in *Escherichia coli* during shake flask cultivation (working volume: 400 mL). The maximum activity for this L-AI was 55.2 µkat_{D-Galactose, 60 °C}/L_{culture}. The enzyme was purified by heat treatment and anion exchange chromatography. The purified enzyme showed maximum activity at 75 °C and pH 5.5 to 7, while about 70% of activity was retained at pH 4 (Fig. 1). Moreover, it was thermostable at 65 °C for 120 min. The L-AI was completely stable for 72 h in 100 mM sodium acetate buffer (pH 4.5) and MES buffer (pH 5.5 and 6.5) at 4 °C. Furthermore, various metal salts and the metal chelating compound EDTA (ethylenediaminetetraacetic acid) were tested for their influence on activity. The L-AI showed maximum activity in the presence of 1.5 mM CoCl₂.

Isomerization of galactose in sweet or sour whey requires a L-AI that has high activity and stability at a pH below pH 6.7. The L-AI from *Lentilactobacillus parakefiri* DSM 10551 has a broad pH profile (Fig. 1) and high activity compared to other known L-AI reported in the literature and applied in industrial production of tagatose (cf. Table 1).

**Table 1: Overview of the effects of pH on the activity of various L-AI**

| **Source of L-AI** | **pH optimum** | **EA pH 6.5 [U/mg]** | **EA pH 4.5 [U/mg]** |
|---|---|---|---|
| *Lentilactobacillus parakefiri* | 5.5 - 7 | 11.4 | 8.0 |
| *Geobacillus stearothermophilus* | 7 - 7.5 | ≈ 5.0 | no activity |
| *Thermotoga neapolitana* | 7 | NR | NR |
| *Enterococcus faecium* | 4.5 - 5.5 | ≈ 0.3 | ≈ 0.4 |
| *Lactobacillus fermentum* | 6.5 | 9.8 | ≈ 1.2 |
| *Lactobacillus plantarum* | 7.5 | ≈ 6.3 | ≈ 1.3 |
| *Lactobacillus reuteri* | 6 | ≈ 9.9 | ≈ 1.3 |
| *Lactobacillus brevis* | 7 | ≈ 1.2 | ≈ 0.5 |

NR, not reported; One unit (U) of enzyme activity (EA) was defined as the amount of enzyme catalyzing the formation of 1 µmol tagatose min⁻¹ at optimum temperature.

### Example 2:

### Bioconversion of lactose using Lentilactobacillus parakefiri DSM 10551 L-AI

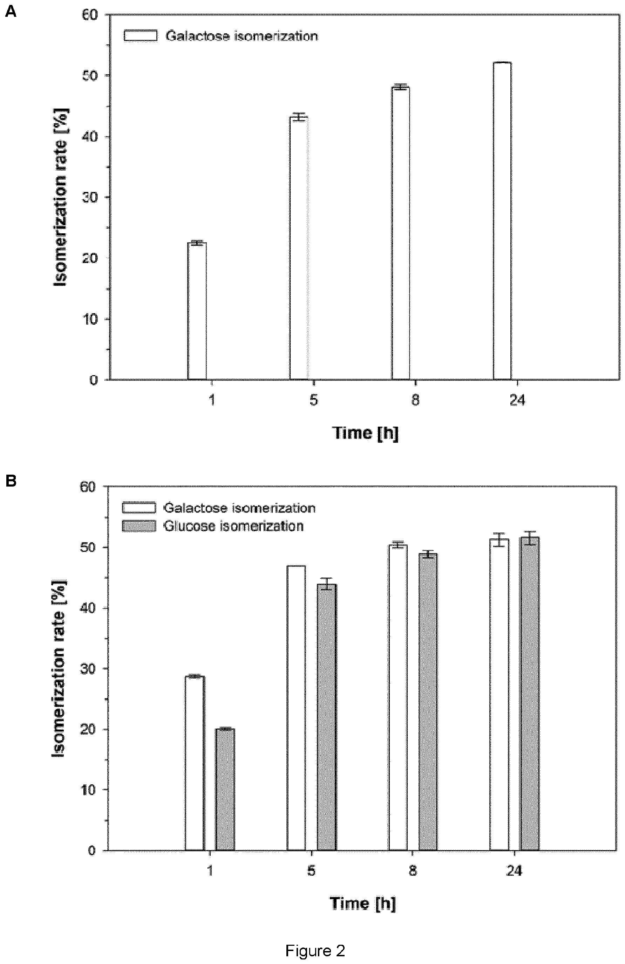

A bi-enzymatic bioconversion of 95 g/L lactose in skim milk ultrafiltration permeate pH 4.5 was done. First, lactose was completely hydrolyzed by a β-galactosidase preparation. After that, galactose was isomerized by *Lentilactobacillus parakefiri* L-AI at 60 °C. After 24 h, an isomerization rate of 52% for tagatose was obtained (Fig. 2 A). However, glucose was not isomerized, because there is no suitable glucose isomerase preparation for isomerization at pH 4.5 on the market.

In addition, a tri-enzymatic bioconversion of 95 g/L lactose in skim milk ultrafiltration permeate pH 6.5 was done. After complete lactose hydrolysis, galactose and glucose were simultaneously isomerized by *Lentilactobacillus parakefiri* L-AI and a glucose isomerase preparation at 60 °C. After 24 h, isomerization rates of 51 and 52 % for tagatose and fructose were obtained, respectively (Fig. 2 B). In conclusion, starting from 95 g/L lactose in skim milk ultrafiltration permeate pH 4.5 and 6.5, 26 g/L of tagatose can be obtained.

The present invention relates to the following amino acid and nucleotide sequences:
SEQ ID NO: 1
   Amino acid sequence of L-arabinose isomerase from *Lentilactobacillus parakefiri* DSM 10551. 
MLQVPDYEFWFVTGSQHLYGEEQLKSVEKDARDMVDKLNASKKLPYPIVFKMVATTADS
ITQLMKDVNYNDKVAGVITWMHTFSPAKNWIRGTKLLQKPLLHLATQYLDHIPYDTIDF
DYMNLNQSAHGDREYGFINARLQKHNKIVYGWWGDPEVQDEIADWEDVAVAYDESFKIK
VARFGDNMRNVAVTEGDKVEAQIQFGWTVDYFALGDLVESVNAVSESDIDKKYKELQDK
YEFVQGDNDKDKYEHSVRYQIREYFGIKNFLDKGNYSAFTTNFEDLYGLEQLPGLAAQL
LMDEGYGFGAEGDWKTAALDRLVKILTHNQATAFMEDYTLELQKGKEAILGSHMLEVDP
GIASDKPRVEVHPLDIGGKADPARLVFTGREGDAMDITISDFGTEYRMIGYAVEGHKAP
KPTPHLPVAKQMWTPKMGLKHGATQWIHDGGGHHTVLTFAANETQIQDLATMFGLTFND
IK
SEQ ID NO: 2
   Native DNA sequence encoding *Lentilactobacillus parakefiri* DSM 10551 L-arabinose isomerase. 
atgttacaagtacctgattatgaattttggtttgttactggtagtcaacatctttacgg
agaagaacaattaaagtccgttgaaaaagatgcccgagacatggttgataagttgaatg
cttcaaagaagcttccttacccaatcgtgttcaagatggttgccaccactgctgacagc
atcactcagttaatgaaagatgttaactacaatgataaagtagccggtgttattacttg
gatgcacacattctcaccagctaaaaactggatccgtggtactaagttattgcaaaagc
cattacttcacttggcaactcaatacttggatcacattccatatgacactatcgacttt
gactacatgaaccttaaccaaagtgctcatggtgaccgtgaatacggctttatcaatgc
tcgtcttcaaaaacacaacaagatcgtttacggctggtggggagatccagaagtccaag
acgaaattgctgactgggaagatgtcgcagttgcttatgacgaaagcttcaagatcaag
gttgctcgtttcggtgataacatgcgtaacgttgctgttactgaaggtgacaaagttga
agctcaaatccaatttggctggaccgttgattacttcgcacttggtgacttggtagaat
cagttaacgctgtttctgaaagtgacattgacaagaagtacaaagaattacaagacaag
tatgaatttgtacaaggcgacaatgacaaagacaagtacgaacattcagttcgttacca
aatccgtgaatacttcggaatcaagaacttcttggacaaaggtaactactcagcattca
ctaccaacttcgaagacctttatggtttggaacaattacctggtcttgcagctcaattg
cttatggacgaaggttatggtttcggtgccgaaggtgactggaagacagccgctcttga
ccgcttagttaagatcttgactcataatcaagctactgcattcatggaagactacactc
ttgaattacaaaagggtaaggaagctatccttggatcacacatgcttgaagttgaccct
ggtattgccagtgacaagcctcgtgttgaagttcacccattggacatcggtggtaaagc
tgatcctgcacgtctggtatttactggtcgtgaaggcgacgcaatggacatcaccattt
ctgacttcggtactgaataccgtatgatcggatatgctgttgaaggacacaaagctcca
aagccaactccacatcttccagttgcaaaacaaatgtggactccaaagatgggcttgaa
gcatggtgctactcaatggattcatgatggtggtggacaccacactgtcttgacatttg
ctgctaacgaaactcaaatccaagacttagctactatgtttggattgacatttaacgac
atcaagtaa
SEQ ID NO: 3
   DNA sequence encoding *Lentilactobacillus parakefiri* DSM 10551 L-arabinose isomerase optimized for expression in *Escherichia coli.*

atgctgcaggttccggattatgaattttggtttgttaccggtagccagcatctgtatgg
tgaagaacagctgaaaagcgttgaaaaagatgcacgtgatatggtggataaactgaacg
caagcaaaaaactgccgtatccgattgtgtttaaaatggttgccaccaccgcagatagc
attacccagctgatgaaagatgtgaactataacgataaagtggcaggcgttattacctg
gatgcatacctttagtccggcaaaaaattggattcgtggcaccaaactgctgcagaaac
cgctgctgcatctggcaacccagtatctggatcatattccgtatgataccatcgacttc
gattacatgaatctgaatcagagcgcacatggtgatcgtgaatatggttttatcaatgc
acgcctgcagaagcacaacaaaattgtttatggttggtggggtgatccggaagttcagg
atgaaattgcagattgggaagatgttgcagttgcctatgatgaaagcttcaaaatcaaa
gttgcccgttttggtgataacatgcgtaatgttgccgttaccgaaggtgataaagttga
agcacagattcagtttggttggaccgttgattattttgcactgggtgatctggttgaaa
gcgttaatgcagttagcgaaagcgatatcgacaagaaatacaaagaactgcaggacaaa
tatgaattcgtgcagggcgataacgacaaagataaatatgaacattcagtgcgctatca
gatccgcgaatattttggcattaaaaactttctggacaaaggcaactatagcgccttta
ccaccaattttgaagatctgtacggtctggaacagctgcctggtctggcagcacagctg
ctgatggatgaaggttatggttttggtgcagaaggtgattggaaaaccgcagcactgga
tcgtctggttaaaattctgacccataatcaggcaaccgccttcatggaagattacaccc
tggaactgcagaaaggtaaagaagcaattctgggtagccacatgctggaagttgatccg
ggtattgcaagcgataaaccgcgtgttgaagttcatccgctggatattggtggcaaagc
agatccggcacgtctggtgtttaccggtcgtgaaggtgatgcaatggatattaccatta
gcgattttggcaccgaatatcgcatgattggttatgcagttgaaggtcataaagcaccg
aaaccgacaccgcatctgccggttgcaaaacaaatgtggaccccgaaaatgggtctgaa
acatggtgcgacccagtggattcatgatggtggtggtcatcataccgttctgacctttg
cagcaaatgaaacccagattcaggatctggcgaccatgtttggcctgacctttaatgat
atcaaataa

## Claims

1. A method for the production of D-tagatose, comprising the steps of:
(a) providing a solution containing D-galactose;
(b) adding an enzyme having L-arabinose isomerase activity to the solution provided in step (a), wherein said enzyme comprises
(i) the amino acid sequence of SEQ ID NO: 1; or
(ii) an amino acid sequence having at least 70% sequence identity to SEQ ID NO: 1 and having L-arabinose isomerase activity; and
(c) incubating the solution with the added enzyme having L-arabinose isomerase activity under conditions and for a duration of time allowing for the enzymatic isomerization of the D-galactose to D-tagatose by said enzyme having L-arabinose isomerase activity.

2. The method of claim 1, wherein step (a) comprises the steps of:
(α-1) providing a solution containing lactose;
(α-2) adding an enzyme having β-galactosidase activity to the solution provided in step (α-1); and
(α-3) incubating the solution with the added enzyme having β-galactosidase activity under conditions and for a duration of time allowing for the enzymatic cleavage of the lactose into D-glucose and D-galactose by said enzyme having β-galactosidase.

3. The method of claim 1 or claim 2, wherein steps (α-3) and/or (c) are performed at an acidic pH.

4. The method according to claim 3, wherein the acidic pH is a pH in the range of from about pH 4.5 to about pH 6.9.

5. The method according to claim 2 or claim 3, wherein step (α-3) is performed at a temperature in the range of from about 5 °C to about 40 °C.

6. The method according to any one of claims 1 to 5, wherein step (c) is performed at a temperature in the range of from about 55 °C to about 70 °C.

7. The method according to any one of claims 2 to 6, wherein the solution containing lactose provided in step (α-1) is whey or a solution generated from whey.

8. The method according to claim 7, wherein the whey is sour whey.

9. The method according to any one of claims 1 to 8, further comprising the step of isolating D-tagatose from the solution obtained in step (c).

10. The method according to any one of claims 1 to 9, wherein the enzyme having L-arabinose isomerase activity consists of
(i) the amino acid sequence of SEQ ID NO: 1; or
(ii) an amino acid sequence having at least 70% sequence identity to SEQ ID NO: 1 and having L-arabinose isomerase activity.

11. The method according to any one of claims 1 to 10, wherein the amino acid sequence in (ii) has at least 72%, at least 74%, at least 76%, at least 78%, at least 80%, at least 82%, at least 84%, at least 86%, at least 88%, at least 90%, at least 91%, at least 92%, at least 93%, at least 94%, at least 95%, at least 95.5%, at least 96%, at least 96.5%, at least 97%, at least 97.5%, at least 98%, at least 98.2%, at least 98.4%, at least 98.6%, at least 98.8%, at least 99%, at least 99.1%, at least 99.2%, at least 99.3%, at least 99.4%, at least 99.5%, at least 99.55%, at least 99.6%, at least 99.65%, at least 99.7%, at least 99.75%, at least 99.8%, at least 99.85%, at least 99.9%, at least 99.91%, at least 99.92%, at least 99.93%, at least 99.94%, at least 99.95%, at least 99.96%, at least 99.97%, at least 99.98%, or at least 99.9% sequence identity to SEQ ID NO: 1.

12. The method according to any one of claims 1 to 11, wherein the enzyme having L-arabinose isomerase activity consists of the amino acid sequence of SEQ ID NO: 1.

13. A product containing D-tagatose, wherein said product is obtainable by the method according to any one of claims 1 to 12.

14. The product according to claim 13, wherein the product is selected from the group consisting of foodstuffs, feedstuffs, pharmaceuticals, and food supplements.

15. The product according to claim 13 or claim 14, wherein the product is a sweetener or a sugar syrup.
